# EUROPEAN PATENT SPECIFICATION

(11) **EP 2 905 818 B1**
(45) Date of publication and mention of the grant of the patent: **20.12.2023**
(21) Application number: 13843622.5
(22) Date of filing: 03.10.2013
(51) Int. Cl.: H01L 33/50

(54) **WHITE-LIGHT EMITTING DEVICE, LIGHTING DEVICE, AND LIGHTING DEVICE FOR DENTISTRY**
WEISSLICHTEMITTIERENDE VORRICHTUNG, BELEUCHTUNGSVORRICHTUNG UND BELEUCHTUNGSVORRICHTUNG FÜR DIE ZAHNMEDIZIN
DISPOSITIF D'ÉMISSION DE LUMIÈRE BLANCHE, DISPOSITIF D'ÉCLAIRAGE, ET DISPOSITIF D'ÉCLAIRAGE POUR LA DENTISTERIE

(30) Priority: 04.10.2012 JP 2012222238; 17.10.2012 JP 2012230083; 30.10.2012 JP 2012239653
(43) Date of publication of application: 12.08.2015
(60) Divisional application: 16204993.6
(73) Proprietor: Seoul Semiconductor Co., Ltd., Ansan-si, Gyeonggi-do 15429 (KR)
(72) Inventor: YAMAKAWA, Masahiko, Tokyo 105-8001 (JP); NAKAGAWA, Katsutoshi, Tokyo 105-8001 (JP); SHIRAKAWA, Yasuhiro, Tokyo 105-8001 (JP); OOYA, Yasumasa, Tokyo 105-8001 (JP); OKAMURA, Masami, Tokyo 105-8001 (JP)
(74) Representative: Hoffmann Eitle
(86) International application number: PCT/JP2013/005912
(87) International publication number: WO 2014/054290

(56) References cited:
- WO-A1-2010/147005
- WO-A1-2012/029305
- WO-A1-2012/108065
- JP-A- 2002 344 021
- JP-A- 2006 008 862
- JP-A- 2006 049 799
- JP-A- 2006 063 233
- JP-A- 2009 117 160
- JP-A- 2010 092 993
- KR-B1- 100 362 064
- US-A1- 2006 027 781

## Description

### TECHNICAL FIELD

The present invention relates to a white light emitting device adapted to a dental lighting system and a dental lighting system.

### BACKGROUND

In recent years, a white light emitting device using a light-emitting diode (LED) has been noticed from viewpoints of a countermeasure for energy saving and carbon dioxide emission reduction. For example, when it is compared with a conventional incandescent lamp using a tungsten filament, the LED has a longer operating life, and energy saving is possible. In a conventional white light emitting device, white light is enabled by exciting a YAG phosphor by using a blue LED having a light emission intensity peak at a wavelength region of, for example, 400 nm or more and 530 nm or less, and by mixing blue light of the blue LED and yellow light of the YAG phosphor.

The white light emitting device using the LED is widely used as a traffic light, a backlight of a liquid crystal display device, and further as a general lighting equipment such as an interior light. In a light emission spectrum of the conventional white light emitting device using the blue LED, a peak height of the blue light of the blue LED is high to be 1.5 times or more as high as a peak height of the yellow light in the phosphor, and there is a tendency in which an effect of the blue light is strong.

On the other hand, requirements demanded for the white light emitting device become various in accordance with popularization of the white light emitting device using the LED. For example, there have been problems in the white light emitting device where the blue LED and the YAG phosphor are combined such that an object looks yellow-tinged depending on a looking direction, or color shading of blue or yellow occurs.

To solve the problems as stated above, for example, a white light emitting device in which an ultraviolet light-emitting diode and phosphors are combined is proposed. According to a white light emitting device in which the ultraviolet light-emitting diode and three kinds of phosphors of a blue phosphor, a green phosphor and a red phosphor are combined, a high color rendering property is enabled.

In the conventional white light emitting device, it is set such that a light emission intensity peak of red is to be high to enable the high color rendering property. When an object is lighted by the white light emitting device having a light emission spectrum as stated above, a color tone of the object to be lighted (the object where the light is irradiated) looks clear. On the other hand, in case of a cloth and so on, there is a case when it is felt that a color tone lighted by the white light emitting device with high color rendering property and a color tone under sunlight are different. Namely, there is a problem in which a difference in colors is felt for the same object to be lighted depending on the case when the light radiated from the white light emitting device with high color rendering property is irradiated and the case when the sunlight is irradiated.

In the white light emitting device using the blue LED, it is reported that the light is glaring more than necessary, there is a negative effect on a human body in a circadian rhythm and so on, or the like because the light emission of the blue LED is too strong. As stated above, various problems occur resulting from popularization of the white light emitting device using the blue LED.

A dental lighting system can be cited as one of the white light emitting devices. There are various treatment methods as dental treatments such as an oral cavity treatment such as a treatment of decayed teeth and a treatment of gums, an aesthetic treatment such as teeth whitening (so-called whitening) for beauty. A certain color rendering property to light in a mouth is required for the dental lighting system, but the color rendering property is insufficient in a lighting system including the conventional white light emitting device using the blue LED, and therefore, there has been a problem that a color tone in the mouth cannot be accurately grasped. Accordingly, various dental lighting systems have been developed as the white LED light with high color rendering property.

Further, there is a treatment using a specialized dental resin as one kind of the dental treatments. The dental resin is coated after the treatment, then the dental resin is cured to thereby protect a treated part. When there is a gap between the treated part and the cured dental resin, possibilities in which hypersensitivity, a micro-leakage, an enamel edge chip, a secondary decayed tooth, and so on are incurred become high. Therefore, when the dental resin before cured is coated on the treated part, it is necessary to coat without any gap, and thereafter to be cured.

A light-curing resin which is cured by light at a wavelength of 380 nm or more and 450 nm or less is generally used for the dental resin. In the conventional dental resin, it is cured early when the blue LED light is strong, and the problem that the gap is generated between the treated part and the cured dental resin has been occurred. Besides, the similar problem has occurred as for the white light emitting device with high color rendering property.

Furthermore, US 2006/027781 A1 is concerned with phosphor systems for a white LED. The phosphor systems are excited by a non-visible to near-UV radiation source having an excitation wavelength ranging from about 250 to 420 nm and comprise one to four phosphors. According to an embodiment, a white light LED was constructed by mixing a phosphate-based yellow phosphor with an aluminate-based blue phosphor, and exciting the two-phosphor mixture with a radiation source that provided excitation radiation ranging from about 370 to 400 nm, that is, non-visible radiation The phosphate-based yellow phosphor was (Sr_{0.8}Mn_{0.16}Eu_{0.04})₂P_{2.02}O₇, and the aluminate-base blue phosphor was Sr_{0.2}MgEu_{0.8}Al₁₀O₁₇.

KR 10-0362064 B1 inter alia relates to an illumination method for a dental camera. A body of the dental camera is formed by coupling a head, a cylindrical upper case, and a cylindrical lower case. An LED (Light Emitting Diode) is installed at an end portion of the cylindrical lower case and connected to an optical fiber through a connector.

WO2012/108065A1 is considered the most relevant prior art and is concerned with a white light source for general lighting purposes and which is supposed to reproduce the light emission spectrum of natural light According to an embodiment, a bulb-type white light emitting device is provided, which contains a plurality of LED chips emitting a first light at 400 nm. The white light source contains a phosphor layer including five different phosphors, which emit second lights at peak wavelengths of 445 nm, 490 nm, 530 nm, 555 nm and 630 nm, respectively. The resulting white light has a color temperature of 5100 K.

### RELEVANT REFERENCES

### Patent Reference

Reference 1: JP-A 10-242513
Reference 2: WO 2007/037120 A1
Reference 3: JP-A 2008-540542
Reference 4: JP-B 4862098

### SUMMARY

A problem to be solved by the present invention is to improve at least one of the following: glare which is felt resulting that a light intensity at a blue visible region is strong, an effect on a circadian rhythm, and an effect on a dental treatment.

According to the present invention, the problem is solved by a white light emitting device adapted to a dental lighting system as defined in claim 1.

A white light emitting device according to the present invention is a white light emitting device adapted to a dental lighting system, which comprises a light-emitting diode emitting first light having a light emission intensity peak at a wavelength region of from 350 to 490 nm; and a phosphor layer emitting white light in response to excitation by the first light of the light-emitting diode. The phosphor layer includes a plurality of phosphors which emit second lights by being excited by the first light, the second lights have light emission intensity peaks different from one another at a wavelength region of from 420 to 700 nm, and the white light is formed by the second lights, or the first light and the second lights. Among a light emission spectrum of the light-emitting diode and each of the light emission spectra of the plurality of phosphors, it has at least one region where two or more light emission spectra are overlapped. A relative color temperature of the white light is 2500 K or more and 7000 K or less, and, in a light emission spectrum of the white light, when an arbitrary local maximum value of a light emission intensity of the local maximum values existing in the wavelength region of from 350 to 780 nm of the white light is set to be "1", and a ratio of a local minimum value of the light emission intensity which is in closest proximity to said arbitrary local maximum value at a long wavelength side relative to said arbitrary local maximum value is set to an intensity ratio, all of the intensity ratios are 0.5 or more.

### BRIEF DESCRIPTION OF THE DRAWINGS

[Fig. 1] Fig. 1 is a view illustrating an example of a white light emitting device according to an embodiment.
[Fig. 2] Fig. 2 is a view illustrating an example of a lighting system of the embodiment.
[Fig. 3] Fig. 3 is a view illustrating an example of the lighting system of the embodiment.
[Fig. 4] Fig. 4 is a view illustrating a light emission spectrum of a white light emitting device of an example 1-1.
[Fig. 5] Fig. 5 is a view illustrating a light emission spectrum of a white light emitting device of an example 1-2.
[Fig. 6] Fig. 6 is a view illustrating a light emission spectrum of a white light emitting device of an example 1-3.
[Fig. 7] Fig. 7 is a view illustrating a light emission spectrum of a white light emitting device of an example 1-4.
[Fig. 8] Fig. 8 is a view illustrating a light emission spectrum of a white light emitting device of an example 1-5.
[Fig. 9] Fig. 9 is a view illustrating a light emission spectrum of a white light emitting device of an example 1-6.
[Fig. 10] Fig. 10 is a view illustrating a light emission spectrum of a white light emitting device of an example 1-7.
[Fig. 11] Fig. 11 is a view illustrating a light emission spectrum of a white light emitting device of an example 1-8.
[Fig. 12] Fig. 12 is a view illustrating a light emission spectrum of a white light emitting device of an example 1-9.
[Fig. 13] Fig. 13 is a view illustrating a light emission spectrum of a white light emitting device of an example 2-1.
[Fig. 14] Fig. 14 is a view illustrating a light emission spectrum of a white light emitting device of an example 2-2.
[Fig. 15] Fig. 15 is a view illustrating a light emission spectrum of a white light emitting device of an example 2-3.
[Fig. 16] Fig. 16 is a view illustrating a light emission spectrum of a white light emitting device of an example 2-4.
[Fig. 17] Fig. 17 is a view illustrating a light emission spectrum of a white light emitting device of a comparative example 2-1.
[Fig. 18] Fig. 18 is a view illustrating a light emission spectrum of a white light emitting device of a comparative example 2-2.
[Fig. 19] Fig. 19 is a view illustrating a light emission spectrum of a white light emitting device of a comparative example 2-3.
[Fig. 20] Fig. 20 is a view illustrating a light emission spectrum of a conventional white light emitting device.
[Fig. 21] Fig. 21 is a view illustrating a light emission spectrum of a conventional white light emitting device.

### DETAILED DESRIPTION

### (First Embodiment)

A white light emitting device adapted to a dental lighting system of a present embodiment includes a light-emitting diode and a phosphor layer which emits white light in response to excitation by light of the light-emitting diode.

The light-emitting diode has a peak of a light emission intensity (light emission peak wavelength) at a wavelength region of from 350 to 490 nm (350 nm or more and 490 nm or less). As the light-emitting diode, it is possible to use the light-emitting diode of so-called an ultraviolet light emission, a violet light emission or a blue light emission. The light-emitting diode of the ultraviolet light emission, the violet light emission or the blue light emission is suitable to excite phosphors. Further, it is possible to obtain white light by using the ultraviolet light emission, violet light emission or blue light emission light-emitting diode and various phosphors.

When an arbitrary local maximum value of a light emission intensity at a wavelength region of from 350 to 780 nm (350 nm or more and 780 nm or less) of the white light is set to be "1", a ratio of a local minimum value of the light emission intensity which is in closest proximity to the local maximum value at a long wavelength side relative to the local maximum value (a peak height of the local minimum value/ a peak height of the local maximum value) is 0.5 or more. It is possible to measure a light emission spectrum of a white light emitting device by a total luminous flux measurement using an integrating sphere based on JIS-C-8152.

The local maximum value of the light emission intensity (hereinafter, referred to as the local maximum value) is a maximum value at a part where the light emission spectrum shows a mount shape. Namely, the local maximum value is a maximum value at the mount shape which rises and then falls. The local minimum value of the light emission intensity (hereinafter, referred to as the local minimum value) is a minimum value at a part where the light emission spectrum shows a valley shape. Namely, the local minimum value is a minimum value at the valley shape which falls and then rises. Accordingly, when the value continues to gently rise (including a parallel state), namely, when there is no falling part, it is not called as the local maximum value. When the value continues to gently fall (including the parallel state), namely, when there is no rising part, it is not called as the local minimum value.

The ratio of the local minimum value which is in closest proximity to the arbitrary local maximum value at the long wavelength side relative to the local maximum value at the white light is set to be large such as 0.5 or more when the local maximum value is set to be "1", namely, projection and recession of the light emission spectrum of the white light is made small, and thereby, it is possible to remove a wavelength region which is insufficient in a visible region, and to equalize a way how an object color looks to a way how it looks under sunlight (natural light). When the local maximum value exists but the local minimum value which is in closest proximity to the local maximum value at the long wavelength side does not exist, the ratio of the local minimum value relative to the local maximum value is not limited. In other words, when there is the local minimum value which is in closest proximity to the local maximum value relative to the local maximum value in the light emission spectrum, all of the ratios of the local minimum value relative to the local maximum value become 0.5 or more when the local maximum value is set to be "1".

From a viewpoint of making the projection and recession of the light emission spectrum small, the ratio of the local minimum value of the light emission intensity which is in closest proximity to the arbitrary local maximum value at the long wavelength side relative to the local maximum value of the light emission intensity is more preferably 0.7 or more when the local maximum value is set to be " 1" at the wavelength region of from 350 to 780 nm of the white light. Note that an upper limit of the ratio of the local minimum value which is in closest proximity to the local maximum value at the long wavelength side is preferably "1", and is preferably 0.95 or less in consideration of manufacturability.

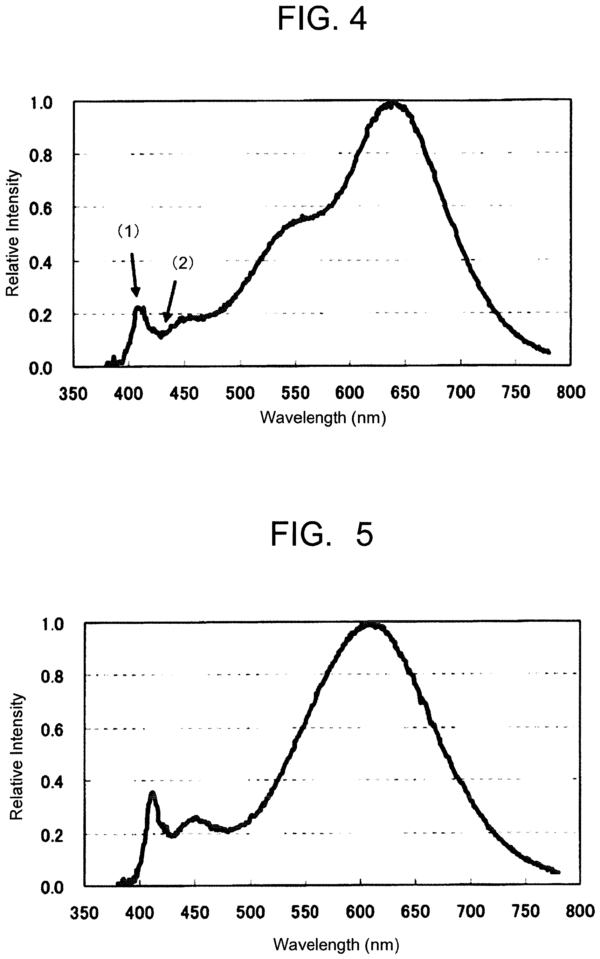

Here, a measurement example of the local maximum value and the local minimum value is illustrated by using Fig. 4. Fig. 4 is a view illustrating a light emission spectrum of a later-described example 1-1. In Fig. 4, a maximum peak intensity exists at a wavelength of 635 nm. At this time, an arbitrary local maximum value becomes a local maximum value (1) (409 nm), and a local minimum value which is in closest proximity to the local maximum value (1) at the long wavelength side becomes a local minimum value (2) (429 nm). When the maximum peak intensity is set to be " 1", a ratio of the local minimum value (2) relative to the local maximum value (1) becomes 0.54 (the light emission intensity of the local minimum value (2) = 0.12/ the light emission intensity of the local maximum value (1) = 0.22). As stated above, the local maximum value of the light emission intensity at the wavelength region of from 350 to 490 nm may be set to be the arbitrary local maximum value.

A color temperature of the white light is 2500 K or more and 7000 K or less. A unit of the color temperature is Kelvin (K). When this color temperature is less than 2500 K or over 7000 K, there is a possibility that it becomes the color temperature which does not exist in the sunlight. Further, the color temperature is more preferably 2700 K or more and 6700 K or less. Note that the color temperature is adjustable by a mixing ratio of the phosphors of respective colors. The color temperature is found by a calculation from the light emission spectrum.

The white light preferably has a maximum peak intensity at a wavelength region of over 490 nm and 780 nm or less. When the maximum peak intensity exists at the wavelength region of over 490 nm and 780 nm or less, it means that the maximum peak intensity does not exist at the wavelength region of from 350 to 490 nm. According to a recent study, it is reported that people in Asia (including Japan) and Europa comparatively feel that the blue light is glaring. It is also reported as for a negative effect on a human body resulting from bath in the blue light for a long time. For example, there are problems such as retinopathy and suppression of secretion of melatonin. It is also reported as for a negative effect on the human body resulting from bath in ultraviolet light for a long time. Accordingly, it is possible to suppress the negative effect on the human body by eliminating the maximum peak intensity between an ultraviolet region and a blue visible region in the light emission spectrum of the white light.

Light emission components from the ultraviolet region to the blue visible region are desirable to be as few as possible. Specifically, it is desirable to be at the same level as components of blue light contained in a spectrum of a black body radiation. The human lives under the sunlight and a flame for a long period of time. It is only for a hundred odd years since an artificial light is accepted in the human life, and familiar light for the human is the sunlight. Therefore, the most desirable light for the human health is the sunlight, and a continuous spectrum of the black body radiation corresponding to the sunlight is desired. In particular, as for a lighting system used in a distance close to a face such as a dental lighting system, it is desired that the light emission spectrum of the white light approximates to a light emission spectrum of the sunlight.

Accordingly, a desirable intensity as for the light emission components from the ultraviolet region to the blue visible region are defined as follows. When a white light spectrum of the white light emitting device and the spectrum of the black body radiation whose color temperature is the same as the white light are compared under an identical visual luminance, a ratio of a maximum light emission intensity of the white light at the wavelength region of from 380 to 490 nm relative to the light emission intensity of the spectrum of the black body radiation is preferably 1.5 or less.

When the above-stated ratio of the intensity is 1.5 or less, it can be regarded as a spectral distribution almost close to the sunlight, and the human does not feel uncomfortable in this light source. The intensity ratio is desirable to be as small as possible. When the intensity ratio is extremely too small, the blue component in the light source becomes small, and the way how the object looks becomes unnatural. Therefore, it is desirable that the blue light is contained for a certain intensity or more in the light source, and a more desirable range of the intensity ratio is 0.8 or more and 1.2 or less.

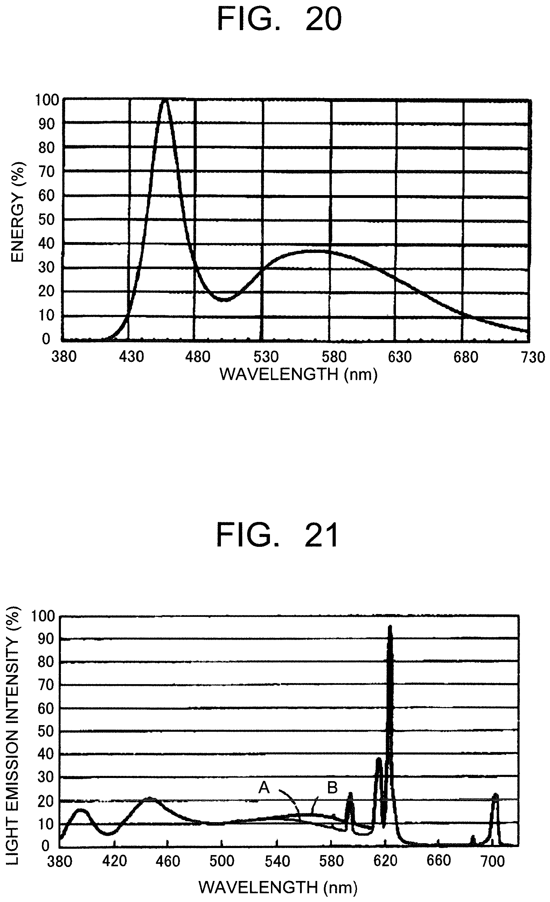

According to the white light emitting device of the embodiment, the projection and recession of the light emission spectrum of the white light emitting device is made small compared to, for example, a light emission spectrum of a conventional white light emitting device illustrated in Fig. 21, and therefore, it is possible to feel a color tone of the object as same as the time when the sunlight is irradiated. Fig. 21 is a view illustrating the light emission spectrum of the conventional white light emitting device in which the ultraviolet light-emitting diode and a blue phosphor, a green phosphor and a red phosphor are combined. Besides, it is possible to prevent an early curing of a dental resin more than necessary by controlling a rate of a light emission spectrum at the blue visible region compared to, for example, a light emission spectrum of a conventional white light emitting device illustrated in Fig. 20. Fig. 20 is a view illustrating the light emission spectrum of the conventional white light emitting device in which the blue light-emitting diode and a YAG phosphor are combined.

### (Second Embodiment)

A white light emitting device of a present embodiment is adapted to a dental lighting system and includes a light-emitting diode and a phosphor layer which emits white light in response to excitation by light of the light-emitting diode.

The light-emitting diode preferably has a peak of a light emission intensity (light emission peak wavelength) at a wavelength region of from 380 to 480 nm, in particular, from 380 to 420 nm. When the peak exists at a wavelength region of less than 380 nm, the ultraviolet light becomes too strong, and there is a possibility that there is a negative effect on the human body when the ultraviolet light leaks out of the phosphor layer. Besides, when the peak exists at a wavelength region of 480 nm over, it is not proper as a light-emitting source of later-described phosphors, and there is a possibility that a color rendering property is lowered. Besides, the light-emitting diode having the light emission peak wavelength at the wavelength region of from 380 to 420 nm is suitable as an excitation source of the phosphors. The light-emitting diode having the light emission intensity peak at the wavelength region of from 380 to 420 nm is used, and thereby, it is easy to adjust the light emission spectrum of the white light.

It is preferable that a ratio of a peak radiant flux (a maximum value of a radiant flux) at a wavelength region of from 430 to 480 nm relative to a radiant flux at a wavelength of 555 nm of the white light is 0.5 or more and 1.3 or less. Further, it is preferable that a ratio of a peak radiant flux at a wavelength region of from 380 to 420 nm relative to the radiant flux at the wavelength of 555 nm of the white light is "0" (zero) or more and 1.3 or less.

An integral value of a radiant flux at a wavelength region of from 380 to 780 nm of the white light is set to be an integral value A, and an integral value of a radiant flux at a wavelength region of from 380 to 480 nm is set to be an integral value B, then a ratio of the integral value B relative to the integral value A (integral value B/ integral value A) is preferably 0.3 or less. When the integral value B/ integral value A is over 0.3, a wavelength component of 380 nm or more and 480 nm or less is too much in the light emission spectrum, and there is a possibility of an effect on the human body and the early curing of the dental resin. The light at the wavelength of 380 nm or more and 480 nm or less is the light from the ultraviolet region to the blue visible region, and the light at the wavelength is necessary for the adjustment of the color temperature and the color rendering property. However, for example, when the light component at the wavelength region of from 380 to 480 nm is too much as the white light emitting device having the light emission spectrum in Fig. 20, a problem of so-called a blue hazard occurs. Besides, the dental resin is easy to be cured by the light at the wavelength region of from 380 to 480 nm, and there is a possibility that it is cured early more than necessary during the dental treatment.

When an integral value of a radiant flux at a wavelength region of from 380 to 429 nm is set to be an integral value C, and an integral value of a radiant flux at a wavelength region of from 430 to 480 nm is set to be an integral value D, a ratio of the integral value C relative to the integral value A (integral value C/ integral value A) is preferably 0.01 or more and 0.12 or less, and a ratio of the integral value D relative to the integral value A (integral value D/ integral value A) is preferably 0.08 or more and 0.25 or less. An integral value (area ratio) at the wavelength region of from 380 to 429 nm (the wavelength region from the ultraviolet region to the violet visible region) and an integral value (area ratio) at the wavelength region of from 430 to 480 nm (the blue visible region) are set to be the above-stated ranges, and thereby, it is possible to adjust the color temperature without lowering the color rendering property. Note that it is possible to measure the light emission spectrum of the white light by, for example, the total luminous flux measurement using the integrating sphere based on JIS-C-8152.

It is preferable to adjust a peak height of the light emission spectrum. For example, when a peak of the radiant flux at the wavelength of 555 nm (peak₅₅₅ₙₘ) is set to be "1", a ratio of a peak radiant flux (peak₄₃₀₋₄₈₀ₙₘ) at the wavelength region of from 430 to 480 nm relative to the peak of the radiant flux at the wavelength of 555 nm (peak₄₃₀₋₄₈₀ₙₘ/ peak₅₅₅ₙₘ) is preferably 0.5 or more and 1.3 or less. A reason why 555 nm is set as a reference is that sensitivity of human eyes for light is called as luminosity and it is defined as the standard spectral luminosity V(λ) at the CIE (Commission Internationale de l'Eclairage), and it is defined that the light at the wavelength of approximately 555 nm can be recognized at a highest sensitivity in the spectral luminous efficiency V(λ) defined by the CIE. The peak radiant flux (peak₄₃₀₋₄₈₀ₙₘ) at the wavelength region of from 430 to 480 nm indicates the peak of the highest light emission intensity among the wavelength region of from 430 to 480 nm. When the peak₄₃₀₋₄₈₀ₙₘ/ peak₅₅₅ₙₘ ratio is less than 0.5, there is a possibility that the color rendering property is lowered and an objected color temperature cannot be obtained because the blue component in the light emission spectrum is insufficient. Besides, when the peak₄₃₀₋₄₈₀ₙₘ/ peak₅₅₅ₙₘ ratio is over 1.3, the blue component in the light emission spectrum is too much, and there are worries that it is felt glare more than necessary and the effect on the circadian rhythm. There is a possibility that the dental resin is early cured. Accordingly, the peak₄₃₀₋₄₈₀ₙₘ/ peak₅₅₅ₙₘ ratio is preferably 0.5 or more and 1.3 or less, and further preferably 0.5 or more and 1.2 or less.

When the peak (peak₅₅₅ₙₘ) of the radiant flux at the wavelength of 555 nm is set to be "1", a ratio of a peak radiant flux (peak₃₈₀₋₄₂₀ₙₘ) at the wavelength region of from 380 to 420 nm relative to the peak of the radiant flux at the wavelength of 555 nm (peak₃₈₀₋₄₂₀ₙₘ/ peak₅₅₅ₙₘ) is preferably "0" (zero) or more and 1.3 or less. The peak radiant flux at the wavelength region of from 380 to 420 nm indicates the peak of the highest light emission intensity in the wavelength region of from 380 to 420 nm. When the peak₃₈₀₋₄₂₀ₙₘ/ peak₅₅₅ₙₘ ratio is "0" (zero), it means that the light emission component at the wavelength region of from 380 to 420 nm does not exist. Besides, when the peak₃₈₀₋₄₂₀ₙₘ/ peak₅₅₅ₙₘ ratio is over 1.3, the light component at the ultraviolet region or the violet visible region is too much, and there are worries that it is felt glare more than necessary and there is the effect on the circadian rhythm. There is a possibility that the dental resin is early cured. Accordingly, the peak₃₈₀₋₄₂₀ₙₘ/ peak₅₅₅ₙₘ ratio is preferably set to be "0" (zero) or more and 1.3 or less, and further preferably "0" (zero) or more and 1.0 or less. The dental resin is cured in accordance with an amount and intensity of light from the ultraviolet region to the blue visible region. It is possible to reduce the glare feeling (sensation of glare) more than necessary and the effect on the circadian rhythm by controlling the integral value (the amount of light) and the intensity (the height of the peak) as the white light emitting device of the embodiment. Further, with the dental lighting system, it is possible to prevent the early curing of the dental resin more than necessary.

In the dental lighting system, a chromaticity range is defined by JIS T5753:2012. When the chromaticity range is converted into a correlated color temperature, the correlated color temperature of the white light emitting device is preferably 3600 K or more and less than 6400 K. The correlated color temperature is a temperature represented by a color (temperature) of a black body radiation which looks closest color to a light source color. A unit of the correlated color temperature is Kelvin (K). The correlated color temperature is a scale representing a light color (blue-tinged and red-tinged) of the light source, and is a value displayed by the color (temperature) of the black body radiation which looks the closest color to the light source. When the correlated color temperature is less than 3600 K, the light color of the light source becomes too red-tinged, and the color rendering property is lowered. On the other hand, when it is 6400 K or more, the light color of the light source is too blue-tinged, and it is easy to be the light emission spectrum where the blue visible region protrudes. Therefore, the correlated color temperature is preferably 3600 K or moe and less than 6400 K, and more preferably 4250 K or more and 5500 K or less. The correlated color temperature is found by a calculation from the light emission spectrum.

An average color rendering index Ra is also defined in JIS5753:2012, and the average color rendering index Ra of the white light emitting device is preferably larger than 85. The color rendering property is a property of the way how the color looks under the light when an object is looked in comparison with the sunlight (it is also referred to as the natural light and the black body radiation). It is the white light emitting device with high color rendering property when the way how the color looks is similar to the way when the object is irradiated by the sunlight. The average color rendering index Ra is a value set as an objective criterion, and is a value representing a color drift generated when a light source to be evaluated lights on a color chip for color rendering evaluation to compare the light source to be evaluated and a defined reference light source, as an index. A maximum value of the average color rendering index Ra is 100, a numeric value becomes small as the color drift of the color rendering property becomes large, and the way how it looks is far from a natural color when it looks under the sunlight. Accordingly, the average color rendering index Ra is preferably larger than 85, further preferably larger than 90. Note that the average color rendering index Ra can be measured by a measurement based on, for example, JIS-Z-8726.

A special color rendering index R9 is preferably 80 or more. In recent years, requirements for an emission color become severe, and the special color rendering indexes R9 to R15 are set in the JIS standard. In the white light emitting device of the embodiment, the special color rendering index R9 is preferably 80 or more. When the special color rendering index R9 is 80 or more, a color tone of red is close to the natural light compared to the reference light source. A reason why the special color rendering index R9 is selected is that it is important to adjust red to improve the color rendering property. Besides, a measurement of the special color rendering index R9 is performed by a method based on JIS-Z-8726.

### (Third Embodiment)

In the present embodiment, examples of phosphors of the white light emitting devices in the first embodiment and the second embodiment are described.

Each of the phosphor layers of the white light emitting devices according to the first embodiment and the second embodiment includes a plurality of phosphors. The phosphor emits light while using light emission of a light-emitting diode as an excitation source. For example, there is also a method in which three kinds of light-emitting diodes of a blue LED, a green LED and a red LED are combined to reproduce the white light, but the light of the light-emitting diode shows a sharp light emission spectrum, and therefore, the human feels glare. In particular, it is not suitable when it is used close to a face such as a dental treatment. It is difficult to have the light emission spectra such as the white light emitting devices in the first embodiment and the second embodiment. The light emission spectrum of the phosphor is a broad light emission spectrum compared to the light emission spectrum of the light-emitting diode. Accordingly, when the white light of the white light emitting device is formed by using the light of the phosphor, it is easy to form the white light having the light emission spectrum as the first embodiment or the second embodiment.

Each of the plurality of phosphors has a peak of a light emission intensity different from one another at a wavelength region of from 420 to 700 nm. For example, the plurality of phosphors preferably have three kinds or more, further four kinds or more of phosphors each having the peak of the light emission intensity different from one another. In particular, when the peak of the light emission intensity of the light-emitting diode is within a range of 350 nm or more and 420 nm or less, it is preferable to use four kinds or more of phosphors. Besides, when the peak of the light emission intensity of the light-emitting diode is within a range of 421 nm or more and 490 nm or less, it is preferable to use three kinds or more of phosphors. As the phosphors, it is preferable to select three kinds, further four kinds from among a blue phosphor, a green phosphor, an yellow phosphor, and a red phosphor.

A half value width of a light emission peak wavelength (the peak of the light emission intensity) of at least one of the plurality of phosphors is preferably 50 nm or more. A phosphor having a broad light emission spectrum such that the half value width of the light emission peak wavelength is 50 nm or more is used, and thereby, for example, it becomes easy to adjust the ratio of the local minimum value of the light emission intensity which is in closest proximity to the arbitrary local maximum value at the long wavelength side relative to the local maximum value of the light emission intensity to be 0.5 or more when the local maximum value is set to be "1". It is possible to obtain the white light which is soft to the human's eyes. When three kinds or more of phosphors each having the different peak of the light emission intensity are used, the half value width of the light emission peak wavelength of at least one kind of phosphor may be 50 nm or more, but it is more preferable that the half value widths of the light emission peak wavelengths of two kinds, further three kinds or more of phosphors are 50 nm or more. An upper limit of the half value width is preferably 100 nm or less.

A plurality of light emission spectra among the light emission spectrum of the light-emitting diode and the respective light emission spectra of the plurality of phosphors have overlapped regions. In the white light emitting device of the first embodiment and the white light emitting device of the second embodiment, it is effective not to make a region which independently protrudes at the visible region, particularly at the wavelength region of from 420 to 700 nm. To that end, it is preferable to have at least one region where two or more light emission spectra are overlapped (for example, the light emission spectra whose peaks of the light emission intensities are adjacent) among the light emission spectrum of the light-emitting diode and the respective light emission spectra of the plurality of phosphors. The number of regions where the light emission spectra are overlapped is preferable to be more such as two regions, further three regions.

Phosphor materials applicable as the phosphors are not particularly limited as long as it is possible to emit visible light by the light of the light-emitting diode. For example, the materials as illustrated below can be cited.

As examples of a blue phosphor (B), there can be cited a europium-activated alkaline earth halophosphate phosphor (a light emission peak wavelength is 440 nm or more and 455 nm re less) and a europium-activated barium magnesium aluminate phosphor (a light emission peak wavelength is 450 nm or more and 460 nm or less). As a blue-green phosphor, there can be cited a europium-activated strontium aluminate phosphor (a light emission peak wavelength is 480 nm or more and 500 nm or less), a europium and manganese-activated barium magnesium aluminate phosphor (a light emission peak wavelength is 510 nm or more and 520 nm or less).

As examples of a green phosphor (G), there can be cited a europium-activated orthosilicate phosphor (a light emission peak wavelength is 520 nm or more and 550 nm or less), a europium-activated β sialon phosphor (a light emission peak wavelength is 535 nm or more and 545 nm or less), and a europium-activated strontium sialon phosphor (a light emission peak wavelength is 510 nm or more and 530 nm or less).

As examples of an yellow phosphor (Y), there can be cited a europium-activated orthosilicate phosphor (a light emission peak wavelength is 550 nm or more and 580 nm or less) and a cerium-activated rare-earth aluminum garnet phosphor (a light emission peak wavelength is 550 nm or more and 580 nm or less).

As examples of a red phosphor (R), there can be cited a europium-activated strontium sialon phosphor (a light emission peak wavelength is 600 nm or more and 650 nm or less), a europium-activated calcium strontium nitride phosphor (a light emission peak wavelength is 610 nm or more and 650 nm or less), a europium-activated lanthanum oxysulfide phosphor (a light emission peak wavelength is 620 nm or more and 630 nm or less), a manganese-activated magnesium florogermanate phosphor (a light emission peak wavelength is 640 nm or more and 660 nm or less), and a europium-activated alkaline earth nitride phosphor (a light emission peak wavelength is 600 nm or more and 650 nm or less).

An average grain diameter of the phosphor is preferably 1 µm or more and 100 µm or less, further preferably 5 µm or more and 50 µm or less. For example, when three kinds or more of phosphors whose peaks of the light emission intensities are different are used, it is necessary to uniformly mix each phosphor, and therefore, the average grain diameter of each phosphor is preferably 1 µm or more and 100 µm or less, further preferably 5 µm or more and 50 µm or less.

### (Fourth Embodiment)

In the present embodiment, structural examples of the white light emitting devices adapted to a dental lighting system according to the first embodiment and the second embodiment, and a dental lighting system including the white light emitting device are described.

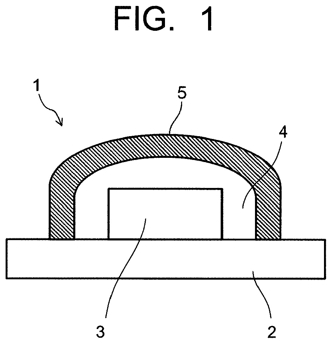

As the white light emitting device of the embodiment, a package-type white light emitting device is illustrated in Fig. 1. Fig. 1 is a view illustrating an example of the white light emitting device of the present embodiment.

A white light emitting device 1 illustrated in Fig. 1 includes an LED chip 3, a base part 2 where the LED chip 3 is mounted, a transparent resin layer 4 provided to cover the LED chip 3, and a phosphor layer 5 further provided on the transparent resin layer 4. A thickness of the transparent resin layer 4 is preferably within a range of 0.01 mm or more and 0.1 mm or less. When a peak of a light emission intensity of the LED chip 3 is at a wavelength region of from 380 to 420 nm, the transparent resin layer 4 is provided, and thereby, it is possible to reduce leakage of ultraviolet light, to reduce an effect on a human body, and to prevent deterioration of peripheral members. The transparent resin layer 4 is not particularly limited, but is preferably a silicone resin. Note that the transparent resin layer 4 may be provided according to need, and for example, when the peak of the light emission intensity of the LED chip 3 is at a wavelength region of from 421 to 480 nm, the transparent resin layer 4 may not be provided.

The phosphor layer 5 is preferably a layer in which a phosphor and a resin are mixed. A method manufacturing the phosphor layer 5 by coating and curing a phosphor paste, a method manufacturing the phosphor layer 5 by covering a molded body in which the phosphor paste is molded into a cap state may be used. A thickness of the phosphor layer 5 is preferably within a range of 0.01 mm or more and 3 mm or less. When the thickness of the phosphor layer 5 is less than 0.01 mm, the phosphor layer 5 is too thin, the light of the LED chip 3 leaks, and the light emission spectrum having an objective peak ratio is difficult to obtain. On the other hand, when the thickness of the phosphor layer 5 is over 3 mm, the light of the LED chip 3 does not uniformly reach an inside of the phosphor layer, and therefore, there is a possibility that fluctuation of emission color occurs.

In Fig. 1, it has a structure in which one phosphor layer is provided for one LED chip (a one-chip type white light emitting device), but it may have a structure in which a plurality of LED chips are covered with the phosphor layer (a multi-chip type white light emitting device).

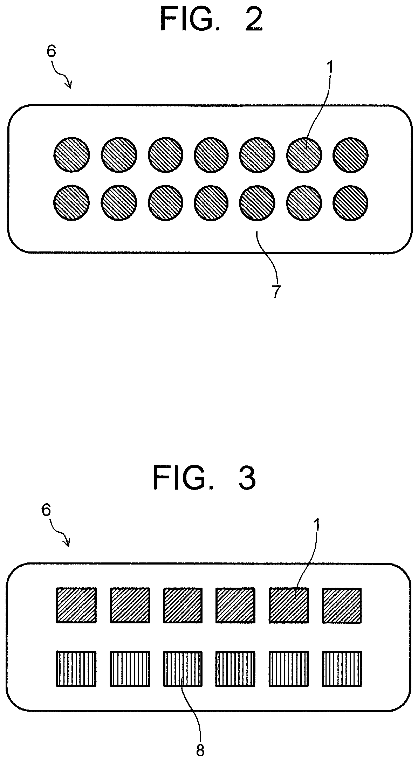

An example of a lighting system is illustrated in Fig. 2. A lighting system 6 illustrated in Fig. 2 includes a base material 7 and the white light emitting devices 1 disposed in plural on the base material 7. As the white light emitting device 1, the white light emitting devices according to the above-stated embodiments are used. Note that other components such as a lens and a cover may be attached according to need. The lighting system 6 is used as a dental lighting system.

Note that in Fig. 2, an example in which a plurality of white light emitting devices are disposed on a flat plate base material is illustrated, but it is not limited thereto. Besides, it may have a structure in which a phosphor layer is provided at an inner surface of a glove as described in Patent Publication No. 4862098 (Patent Document 4). White light emitting devices each having a phosphor layer formed on an inner surface of a glove are provided in plural to be used as a dental lighting system.

Another example of the lighting system is illustrated in Fig. 3. The lighting system 6 illustrated in Fig. 3 includes the white light emitting devices 1 and white light emitting devices 8. As the white light emitting device 1, for example, the white light emitting devices of the above-stated embodiments are used. Besides, as the white light emitting device 8, a white light emitting device showing a light emission spectrum which is different from the white light emitting device 1 can be used. In Fig. 3, the lighting system is constituted by disposing pluralities of white light emitting devices 1 and white light emitting devices 8. Note that depending on a structure of the lighting system, only the white light emitting devices 1 may be provided at the lighting system. Besides, a directivity of light may be adjusted, for example, by disposing a reflector or a lens.

For example, the light emission spectrum of the white light emitting device 8 is set to be the light emission spectrum in which the blue visible region protrudes as illustrated in Fig. 20, and thereby, the white light emitting device 1 which emits the white light having the light emission spectrum whose protrusion of the blue visible region is suppressed and the white light emitting device 8 which emits the white light having the light emission spectrum whose blue visible region protrudes are combined. The white light emitting device to be light-emitted is switched according to an object thereof, and thereby, it is possible to correspond to, for example, both a treatment process and a curing process of a dental resin in a dental treatment by one lighting system.

The white light of the white light emitting device adapted to a dental lighting system and the dental lighting system as stated above has a light emission spectrum whose projection and recession are small which approximates to the light emission spectrum of the sunlight, and therefore, it is not felt glare more than necessary. The color tone looks equivalent to a case when the sunlight is irradiated, and therefore, a color in a mouth looks natural. Accordingly, it is effective for the dental lighting system which is used at a position close to a face. Besides, it is controlled such that there is no peak of the light emission intensity at the light emission spectrum from the ultraviolet region to the blue visible region, and thereby, it is possible to prevent the early curing of the dental resin more than necessary.

The dental lighting system is used at a position close to the human eyes. The dental lighting system is able to light in a mouth. The dental lighting system of the embodiment is used, and thereby, it is possible to clearly look in the mouth. Besides, the projection of the light emission spectrum at the blue visible region is reduced, and therefore, it is possible to prevent the early curing of the dental resin more than necessary in the treatment using the dental resin. Besides, even when the dental lighting system is used at the position close to the human face, it is possible to reduce uncomfortable feeling because it is not felt glare more than necessary.

### EXAMPLES

### <Example 1>

In the present example, concrete examples of the white light emitting device based on the first embodiment are described.

### (Example 1-1)

An LED chip having a light emission peak wavelength of 400 nm was prepared, and disposed on an alumina substrate. Next, as phosphors which emit light by irradiating an electromagnetic wave at a wavelength of 400 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated orthosilicate green phosphor having a light emission peak wavelength of 530 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor is approximately 18 µm. Further, respective phosphors are mixed at a ratio of the blue phosphor: the blue-green phosphor: the green phosphor: the yellow phosphor: the red phosphor = 5: 10: 15: 20: 50 as a weight ratio (mass ratio), then mixed with a transparent resin (silicone resin), and coated on the LED chip where a transparent resin layer (thickness: 0.01 mm) was provided to thereby manufacture a white light emitting device. A correlated color temperature of an emission color of the white light emitting device was 2800 K. Besides, a thickness of a phosphor layer was set to be 0.4 mm.

Among the above-stated phosphors, half value widths of the light emission peak wavelengths of the blue-green phosphor, the green phosphor, the yellow phosphor, and the red phosphor were each 50 nm or more. In light emission spectra of the blue phosphor, the blue-green phosphor, the green phosphor, the yellow phosphor, and the red phosphor, a region where the light emission spectra of the phosphors whose light emission peak wavelengths were adjacent were overlapped was each held.

Next, a light emission spectrum of the white light emitting device of the example 1-1 was measured by the total luminous flux measurement using the integrating sphere based on JIS-C-8152. The measurement result is illustrated in Fig. 4. As it can be seen from Fig. 4, a ratio of a local minimum value which is adjacent to an arbitrary local maximum value at a long wavelength side relative to the local maximum value was each 0.5 or more when the local maximum value is set to be "1".

### (Example 1-2)

An LED chip having a light emission peak wavelength of 400 nm was prepared, and disposed on an alumina substrate. Next, as phosphors which emit light by irradiating an electromagnetic wave at a wavelength of 400 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated β-sialon green phosphor having a light emission peak wavelength of 540 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 13 µm. Further, respective phosphors are mixed at a ratio of the blue phosphor: the blue-green phosphor: the green phosphor: the yellow phosphor: the red phosphor = 10: 5: 15: 20: 50 as a weight ratio (mass ratio), then mixed with a transparent resin (silicone resin), and coated on the LED chip where a transparent resin layer (thickness: 0.05 mm) was provided to thereby manufacture a white light emitting device. A correlated color temperature of an emission color of the white light emitting device was 2800 K. Besides, a thickness of a phosphor layer was set to be 0.2 mm.

Among the above-stated phosphors, half value widths of the light emission peak wavelengths of the blue-green phosphor, the green phosphor, the yellow phosphor, and the red phosphor were each 50 nm or more. In light emission spectra of the blue phosphor, the blue-green phosphor, the green phosphor, the yellow phosphor, and the red phosphor, a region where the light emission spectra of the phosphors whose light emission peak wavelengths were adjacent were overlapped was each held.

Next, a light emission spectrum of the white light emitting device of the example 1-2 was measured by the total luminous flux measurement using the integrating sphere as same as the example 1-1. The measurement result is illustrated in Fig. 5. As it can be seen from Fig. 5, a ratio of a local minimum value which is adjacent to an arbitrary local local maximum value at a long wavelength side relative to the local maximum value was each 0.5 or more when the local maximum value was set to be "1".

### (Example 1-3)

An LED chip having a light emission peak wavelength of 400 nm was prepared, and disposed on an alumina substrate. Next, as phosphors which emit light by irradiating an electromagnetic wave at a wavelength of 400 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated orthosilicate green phosphor having a light emission peak wavelength of 530 nm, a europium-activated orthosilicate yellow phosphor having a light emission peak wavelength of 555 nm, and a europium-activated alkaline earth nitride red phosphor having a light emission peak wavelength of 635 nm were prepared. An average grain diameter of each phosphor was approximately 28 µm.

Further, respective phosphors were mixed at a ratio of the blue phosphor: the blue-green phosphor: the green phosphor: the yellow phosphor: the red phosphor = 5: 5: 15: 25: 50 as a weight ratio (mass ratio), then mixed with a transparent resin (silicone resin), and coated on the LED chip where a transparent resin layer (thickness: 0.1 mm) was provided to thereby manufacture a white light emitting device. A correlated color temperature of an emission color of the white light emitting device was 2700 K. Besides, a thickness of a phosphor layer was set to be 1.0 mm.

Among the above-stated phosphors, half value widths of the light emission peak wavelengths of the blue-green phosphor, the green phosphor, the yellow phosphor, and the red phosphor were each 50 nm or more. Besides, in light emission spectra of the blue phosphor, the blue-green phosphor, the green phosphor, the yellow phosphor, and the red phosphor, a region where the light emission spectra of the phosphors whose light emission peak wavelengths were adjacent were overlapped was each held.

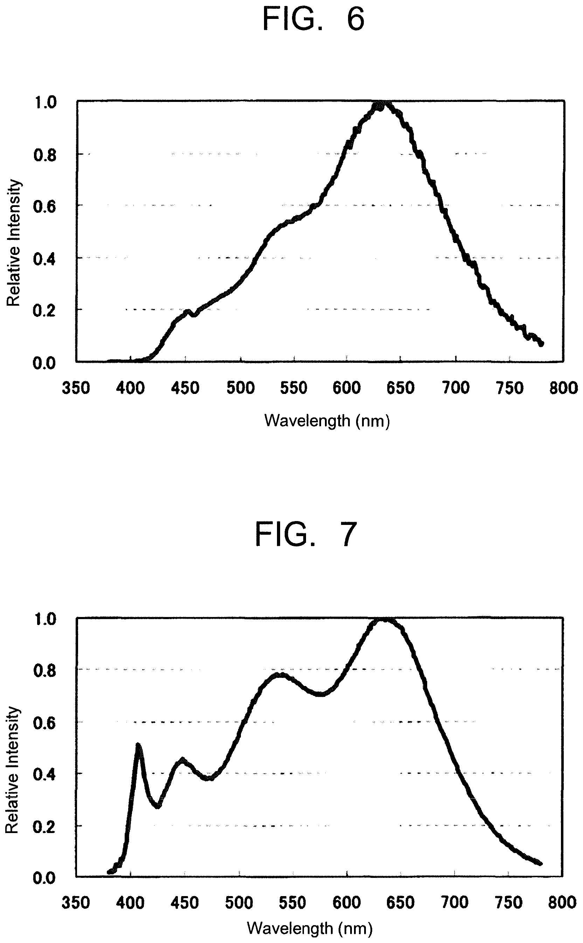

Next, a light emission spectrum of the white light emitting device of the example 1-3 was measured by the total luminous flux measurement using the integrating sphere as same as the example 1-1. The measurement result is illustrated in Fig. 6. As it can be seen from Fig. 6, a ratio of a local minimum value which is adjacent to a local maximum value at a long wavelength side relative to the local maximum value was each 0.5 or more when the local maximum value was set to be " 1".

### (Example 1-4)

An LED chip having a light emission peak wavelength of 400 nm was prepared, and disposed on an alumina substrate. Next, as phosphors which emit light by irradiating an electromagnetic wave at a wavelength of 400 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated orthosilicate green phosphor having a light emission peak wavelength of 530 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 18 µm. Further, respective phosphors were mixed at a ratio of the blue phosphor: the blue-green phosphor: the green phosphor: the yellow phosphor: the red phosphor = 10: 15: 25: 20: 50 as a weight ratio (mass ratio), then mixed with a transparent resin (silicone resin), and coated on the LED chip where a transparent resin layer (thickness: 0.05 mm) was provided to thereby manufacture a white light emitting device. A correlated color temperature of an emission color of the white light emitting device was 3800 K. Besides, a thickness of a phosphor layer was set to be 0.5 mm.

Among the above-stated phosphors, half value widths of the light emission peak wavelengths of the blue-green phosphor, the green phosphor, the yellow phosphor, and the red phosphor were each 50 nm or more. Besides, in light emission spectra of the blue phosphor, the blue-green phosphor, the green phosphor, the yellow phosphor, and the red phosphor, a region where the light emission spectra of the phosphors whose light emission peak wavelengths were adjacent were overlapped was each held.

Next, a light emission spectrum of the white light emitting device of the example 1-4 was measured by the total luminous flux measurement using the integrating sphere as same as the example 1-1. The measurement result is illustrated in Fig. 7. As it can be seen from Fig. 7, a ratio of a local minimum value which is adjacent to an arbitrary local maximum value at a long wavelength side relative to the local maximum value was each 0.5 or more when the local maximum value was set to be "1".

### (Example 1-5)

An LED chip having a light emission peak wavelength of 400 nm was prepared, and disposed on an alumina substrate. Next, as phosphors which emit light by irradiating an electromagnetic wave at a wavelength of 400 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated β sialon green phosphor having a light emission peak wavelength of 540 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 10 µm. Further, respective phosphors were mixed at a ratio of the blue phosphor: the blue-green phosphor: the green phosphor: the yellow phosphor: the red phosphor = 10: 15: 20: 25: 30 as a weight ratio (mass ratio), then mixed with a transparent resin (silicone resin), and coated on the LED chip where a transparent resin layer (thickness: 0.03 mm) was provided to thereby manufacture a white light emitting device. A correlated color temperature of an emission color of the white light emitting device was 4200 K. Besides, a thickness of a phosphor layer was set to be 0.3 mm.

Among the above-stated phosphors, half value widths of the light emission peak wavelengths of the blue-green phosphor, the green phosphor, the yellow phosphor, and the red phosphor were each 50 nm or more. Besides, in light emission spectra of the blue phosphor, the blue-green phosphor, the green phosphor, the yellow phosphor, and the red phosphor, a region where the light emission spectra of the phosphors whose light emission peak wavelengths were adjacent were overlapped was each held.

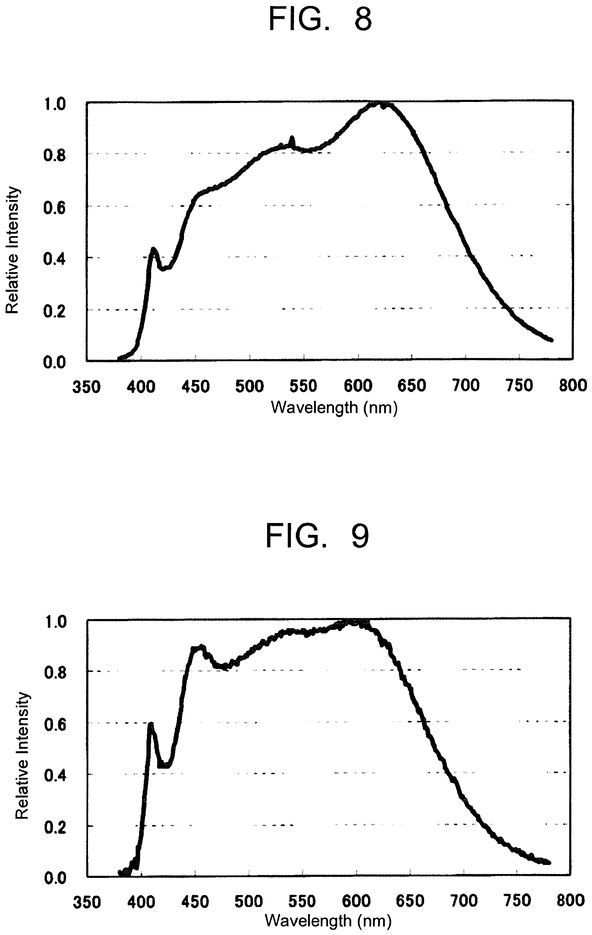

Next, a light emission spectrum of the white light emitting device of the example 1-5 was measured by the total luminous flux measurement using the integrating sphere as same as the example 1-1. The measurement result is illustrated in Fig. 8. As it can be seen from Fig. 8, a ratio of a local minimum value which is adjacent to an arbitrary local maximum value at a long wavelength side relative to the local maximum value was each 0.5 or more when the local maximum value was set to be "1".

### (Example 1-6)

An LED chip having a light emission peak wavelength of 400 nm was prepared, and was disposed on an alumina substrate. Next, as phosphors which emit light by irradiating an electromagnetic wave at a wavelength of 400 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated β sialon green phosphor having a light emission peak wavelength of 540 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 10 µm. Further, respective phosphors were mixed at a ratio of the blue phosphor: the blue-green phosphor: the green phosphor: the yellow phosphor: the red phosphor = 30: 15: 20: 15: 20 as a weight ratio (mass ratio), then mixed with a transparent resin (silicone resin), and coated on the LED chip where a transparent resin layer (thickness: 0.03 mm) was provided to thereby manufacture a white light emitting device. A correlated color temperature of an emission color of the white light emitting device was 5000 K. Besides, a thickness of a phosphor layer was set to be 0.3 mm.

Among the above-stated phosphors, half value widths of the light emission peak wavelengths of the blue-green phosphor, the green phosphor, the yellow phosphor, and the red phosphor were each 50 nm or more. Besides, in light emission spectra of the blue phosphor, the blue-green phosphor, the green phosphor, the yellow phosphor, and the red phosphor, a region where the light emission spectra of the phosphors whose light emission peak wavelengths were adjacent were overlapped was each held.

Next, a light emission spectrum of the white light emitting device of the example 1-6 was measured by the total luminous flux measurement using the integrating sphere as same as the example 1-1. The measurement result is illustrated in Fig. 9. As it can be seen from Fig. 9, a ratio of a local minimum value which is adjacent to an arbitrary local maximum value at a long wavelength side relative to the local maximum value was each 0.5 or more when the local maximum value was set to be "1".

### (Example 1-7)

An LED chip having a light emission peak wavelength of 400 nm was prepared, and disposed on an alumina substrate. Next, as phosphors which emit light by irradiating an electromagnetic wave at a wavelength of 400 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated β sialon green phosphor having a light emission peak wavelength of 540 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 13 µm. Further, respective phosphors were mixed at a ratio of the blue phosphor: the blue-green phosphor: the green phosphor: the yellow phosphor: the red phosphor = 30: 15: 15: 20: 20 as a weight ratio (mass ratio), then mixed with a transparent resin (silicone resin), and coated on the LED chip where a transparent resin layer (thickness: 0.02 mm) was provided to thereby manufacture a white light emitting device. A correlated color temperature of an emission color of the white light emitting device was 5000 K. Besides, a thickness of a phosphor layer was set to be 0.2 mm.

Among the above-stated phosphors, half value widths of the light emission peak wavelengths of the blue-green phosphor, the green phosphor, the yellow phosphor, and the red phosphor were each 50 nm or more. Besides, in light emission spectra of the blue phosphor, the blue-green phosphor, the green phosphor, the yellow phosphor, and the red phosphor, a region where the light emission spectra of the phosphors whose light emission peak wavelengths were adjacent were overlapped was each held.

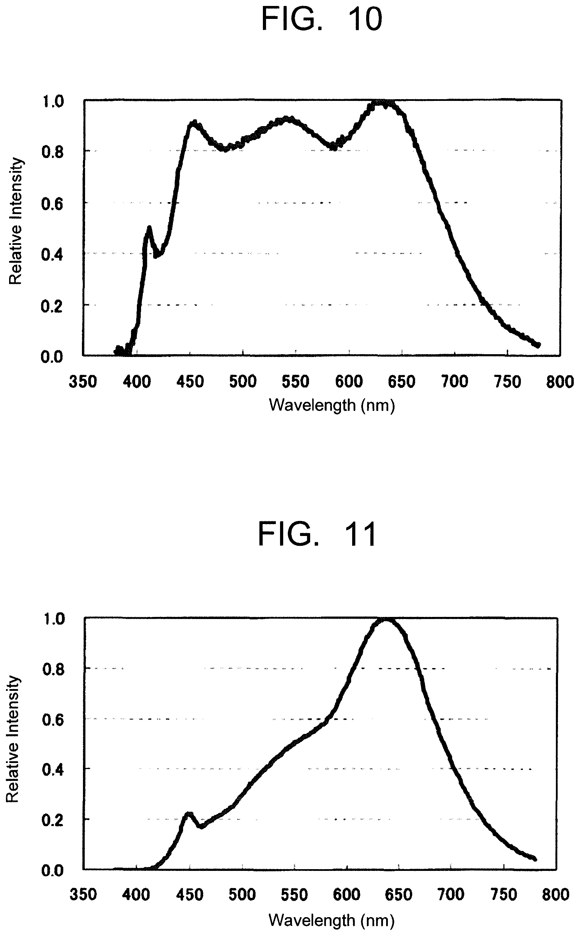

Next, a light emission spectrum of the white light emitting device of the example 1-7 was measured by the total luminous flux measurement using the integrating sphere as same as the example 1-1. The measurement result is illustrated in Fig. 10. As it can be seen from Fig. 10, a ratio of a local minimum value which was adjacent to an arbitrary local maximum value at a long wavelength side relative to the local maximum value was each 0.5 or more when the local maximum value was set to be "1".

### (Example 1-8)

An LED chip having a light emission peak wavelength of 445 nm was prepared, and disposed on an alumina substrate. Next, as phosphors which emit light by irradiating an electromagnetic wave at a wavelength of 445 nm, a europium-activated orthosilicate green phosphor having a light emission peak wavelength of 530 nm, a europium-activated orthosilicate yellow phosphor having a light emission peak wavelength of 530 nm, and a europium-activated strontium sialon red phosphor having a light emission peak wavelength of 630 nm were prepared. Note that an average grain diameter of each phosphor was approximately 17 µm. Next, respective phosphors are mixed at a ratio of the green phosphor: the yellow phosphor: the red phosphor = 20: 30: 50 as a weight ratio (mass ratio), then mixed with a transparent resin (silicone resin), and coated on the LED chip to thereby manufacture a white light emitting device. A correlated color temperature of an emission color of the white light emitting device was 2700 K. Besides, a thickness of a phosphor layer was set to be 0.5 mm.

Among the above-stated phosphors, half value widths of the light emission peak wavelengths of the green phosphor, the yellow phosphor, and the red phosphor were each 50 nm or more. Besides, in light emission spectra of the green phosphor, the yellow phosphor, and the red phosphor, a region where the light emission spectra of the phosphors whose light emission peak wavelengths were adjacent were overlapped was each held.

Next, a light emission spectrum of the white light emitting device of the example 1-8 was measured by the total luminous flux measurement using the integrating sphere as same as the example 1-1. The measurement result is illustrated in Fig. 11. As it can be seen from Fig. 11, a ratio of a local minimum value which was adjacent to an arbitrary local maximum value at a long wavelength side relative to the local maximum value was each 0.5 or more when the local maximum value was set to be "1".

### (Example 1-9)

An LED chip having a light emission peak wavelength of 445 nm was prepared, and disposed on an alumina substrate. Next, as phosphors which emit light by irradiating an electromagnetic wave at a wavelength of 445 nm, a europium-activated orthosilicate green phosphor having a light emission peak wavelength of 530 nm, a europium-activated orthosilicate yellow phosphor having a light emission peak wavelength of 530 nm, and a europium-activated strontium sialon red phosphor having a light emission peak wavelength of 630 nm were prepared. Note that an average grain diameter of each phosphor was approximately 15 µm. Next, respective phosphors were mixed at a ratio of the green phosphor: the yellow phosphor: the red phosphor = 30: 40: 30 as a weight ratio (mass ratio), then mixed with a transparent resin (silicone resin), and coated on the LED chip to thereby manufacture a white light emitting device. A correlated color temperature of an emission color of the white light emitting device was 2700 K. Besides, a thickness of a phosphor layer was set to be 0.4 mm.

Among the above-stated phosphors, half value widths of the light emission peak wavelengths of the green phosphor, the yellow phosphor, and the red phosphor were each 50 nm or more. Besides, in light emission spectra of the green phosphor, the yellow phosphor, and the red phosphor, a region where the light emission spectra of the phosphors whose light emission peak wavelengths were adjacent were overlapped was each held.

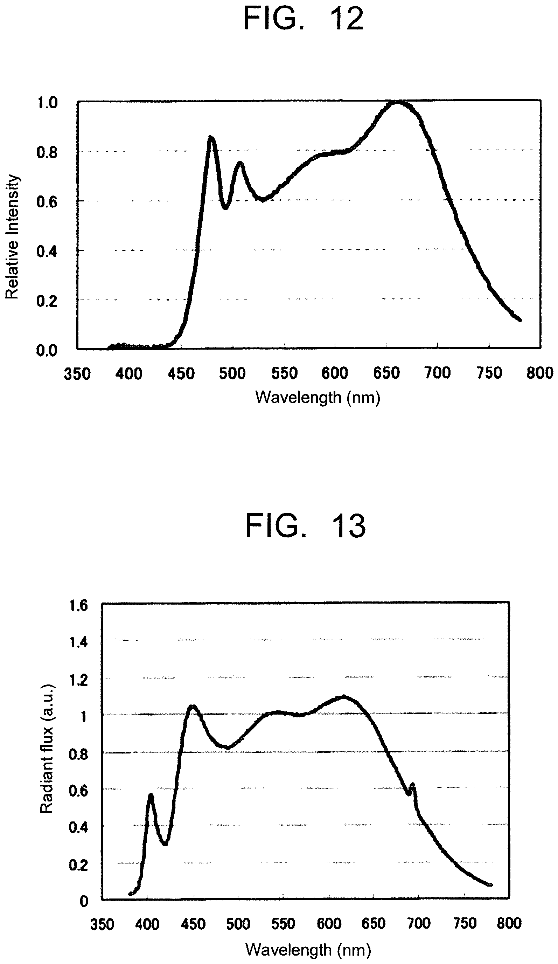

Next, a light emission spectrum of the white light emitting device of the example 1-9 was measured by the total luminous flux measurement using the integrating sphere as same as the example 1-1. The measurement result is illustrated in Fig. 12. As it can be seen from Fig. 12, a ratio of a local minimum value which was adjacent to an arbitrary local maximum value at a long wavelength side relative to the local maximum value was each 0.5 or more when the local maximum value was set to be "1".

Note that in the example 1-1 to example 1-9, each ratio of the local minimum value which is in closest proximity to the arbitrary local maximum value at the long wavelength side when the local maximum value is set to be "1" relative to the local maximum value at the wavelength region of from 350 to 780 nm is illustrated in Table 1.

**[Table 1]**

| | MINIMUM RATIO (LOCAL MINIMUM VALUE/ LOCAL MAXIMUM VALUE) |
|---|---|
| EXAMPLE 1-1 | 0.54 |
| EXAMPLE 1-2 | 0.53 |
| EXAMPLE 1-3 | 0.94 |
| EXAMPLE 1-4 | 0.53 |
| EXAMPLE 1-5 | 0.81 |
| EXAMPLE 1-6 | 0.73 |
| EXAMPLE 1-7 | 0.80 |
| EXAMPLE 1-8 | 0.74 |
| EXAMPLE 1-9 | 0.67 |

### (Comparative Examples 1-1, 1-2)

A one-chip type white light emitting device having the light emission spectrum in Fig. 20 was prepared as a comparative example 1-1, and a one-chip type white light emitting device having the light emission spectrum in Fig. 21 was prepared as a comparative example 1-2.

### (Examples 1-1A to 1-9A, Comparative Examples 1-1A, 1-2A)

A special color rendering index R9 (red) was measured by using each of the white light emitting devices of the example 1-1 to the example 1-9, the comparative example 1-1, the comparative example 1-2. Results thereof are illustrated in Table 2.

**[Table 2]**

| | WHITE LIGHT SOURCE | R9 |
|---|---|---|
| EXAMPLE 1-1A | EXAMPLE 1-1 | 97 |
| EXAMPLE 1-2A | EXAMPLE 1-2 | 96 |
| EXAMPLE 1-3A | EXAMPLE 1-3 | 99 |
| EXAMPLE 1-4A | EXAMPLE 1-4 | 71 |
| EXAMPLE 1-5A | EXAMPLE 1-5 | 83 |
| EXAMPLE 1-6A | EXAMPLE 1-6 | 88 |
| EXAMPLE 1-7A | EXAMPLE 1-7 | 73 |
| EXAMPLE 1-8A | EXAMPLE 1-8 | 82 |
| EXAMPLE 1-9A | EXAMPLE 1-9 | 82 |
| COMPARATIVE EXAMPLE 1-1A | COMPARATIVE EXAMPLE 1-1 | -20 |
| COMPARATIVE EXAMPLE 1-2A | COMPARATIVE EXAMPLE 1-2 | 49 |

Evaluations not only by the average color rendering index Ra but also by a special color rendering index Ri (where "i" is 9 or more and 15 or less) are necessary to make the object look to be the same color tone as the sunlight. A chroma of a color chip used for the special color rendering evaluation becomes high compared to a color chip used for the average color rendering evaluation, and when the evaluation of color such as a color reproducibility is performed, a high chroma region is also necessary, and therefore, it is necessary to use not only the average color rendering index but also Ri together. In the present example, R9 which is representatively used is used from among the special color rendering indexes.

As it can be seen from Table 1 and Table 2, it turned out that the white light emitting devices according to the present examples show excellent properties. Besides, in each of the white light emitting devices of the example 1-1A to the example 1-9A, the special color rendering index R9 was a large value. An object looks at the color tone equivalent to the case when the sunlight is irradiated according to the white light emitting device showing R9 as stated above. Therefore, it is suitable for the dental lighting system which lights in the mouth.

### (Examples 1-10 to 1-20, Comparative Examples 1-3, 1-4)

Next, a test verifying an effect of the obtained light source on a human body is performed as for the manufactured white light emitting devices. As for a test light source, a sensory test is performed whether or not a person feels uncomfortable when the person is exposed under the same light intensity.

The same white light emitting devices as the comparative example 1-1, the comparative example 1-2 were used for the comparative example 1-3, the comparative example 1-4. Besides, white light emitting devices of the example 1-10 to the example 1-20 were manufactured as described below.

An LED chip having a light emission peak wavelength of 380 nm was prepared. Next, as phosphors which emit light by irradiating an electromagnetic wave at a wavelength of 380 nm, a europium-activated alkaline earth halophosphate blue phosphor having a light emission peak wavelength of 445 nm, a europium-activated strontium aluminate blue-green phosphor having a light emission peak wavelength of 490 nm, a europium-activated β sialon green phosphor having a light emission peak wavelength of 540 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 13 µm. Respective phosphors were mixed at a predetermined ratio, then mixed with a silicone resin, and coated on the LED chip where a transparent resin layer (thickness: 0.02 mm) was provided to thereby manufacture a white light emitting device. Note that the mixing ratio of the respective phosphors was changed into various ratios, and thereby, various white light emitting devices whose correlated color temperatures of the emission colors were within a range of 2500 K or more and 7000 K or less were obtained. Color temperatures of each example and each comparative example are illustrated in Table 3. Besides, the example 1-19 is a mixed phosphor which contains the blue phosphor component for a very small amount, and the example 1-20 is a mixed phosphor in which a phosphor component having a light emission peak wavelength at the blue visible region is excluded. Besides, a thickness of a phosphor layer was set to be 0.2 mm.

Light emission spectra of the white light emitting devices of the comparative example 1-3, the comparative example 1-4, and the example 1-10 to the example 1-20 were measured by the total luminous flux measurement using the integrating sphere as same as the example 1-1. Each ratio of a local minimum value which is in closest proximity to an arbitrary local maximum value at a long wavelength side when the local maximum value is set to be "1" relative to the local maximum value at a wavelength region of from 350 to 780 nm is illustrated in Table 3. At all of the light emission spectra of the example 1-10 to the example 1-20, it is verified that each ratio of the local minimum value which is adjacent to the arbitrary local maximum value at the long wavelength side relative to the local maximum value (local minimum value/ local maximum value) is 0.5 or more when the local maximum value is set to be " 1". Besides, maximum peak intensities of all of the light emission spectra of the white light emitting devices according to the example 1-10 to the example 1-20 exist within a range of larger than 490 nm and 780 nm or less.

Next, maximum peak intensities of the light emission spectra at the violet to blue visible region (380 nm or more and 490 nm or less) within the visible light (380 nm or more and 780 nm or less) were measured, and they were compared with an intensity of a black body radiation spectrum at the same wavelength as a wavelength showing the maximum peak intensity within the range of 380 nm or more and 490 nm or less. At this time, the light emission spectra used for the comparison were as follows. When a light emission spectrum of the light source of the embodiment was set to be A(λ), a spectrum of the black body radiation at the same color temperature as the light source of the embodiment was set to be B(λ), a spectrum of a spectral luminous efficiency was set to be V(λ), the spectra of A(λ), B(λ) which satisfy ∫A(λ)· V(λ)dλ = ∫B(λ)· V(λ)dλ were found, and shapes of both spectra of A(λ), B(λ) were compared. At the wavelength region of from 380 to 490 nm of the light emission spectrum A(λ), a wavelength where the spectrum intensity becomes the maximum is set to be λ*, a maximum intensity is set to be A*, and the spectrum intensity of B(λ) at the wavelength λ* is set to be B*, to find an intensity ratio (A*/ B*).

Next, the white light emitting devices of the example 1-10 to the example 1-20, the comparative example 1-3, and the comparative example 1-4 were lighted in front of 100 subjects, to perform the sensory test relating to the way how the light source looks. As the subjects, each of 10 men and women of 10 years old or more and 25 years old or less, 26 years old or more and 40 years old or less, 41 years old or more and 55 years old or less, 56 years old or more and 70 years old or less, 71 years old or more were randomly selected, and they were asked to take the test. The subjects were asked to grade each light source on how it looked into five stages of "comfortable", "not uncomfortable", "feel like uncomfortable", "uncomfortable", "very uncomfortable" to evaluate them.

The light emission properties, the results of the sensory test of the white light emitting devices of the example 1-10 to the example 1-20, the comparative example 1-3, and the comparative example 1-4 are illustrated in Table 3. Note that as description of the results of the sensory test, properties of the rank which were selected the most in the evaluations of 100 persons was described. The number in parenthesis is the number of responders of the rank. Note that in Table 3, a minimum ratio from among the ratio of the local minimum value which is adjacent to the arbitrary local maximum value at the long wavelength side relative to the local maximum value (local minimum value/ local maximum value) was each illustrated.

**[Table 3]**

| | Color temperature (k) | Ratio (local minimum value/ local maximum value) | λ* (nm) | Intensity ratio (A*/B*) | Way how light source looks (the number of persons) |
|---|---|---|---|---|---|
| Example 1-10 | 5000 | 0.55 | 450 | 1.70 | Uncomfortable (45) |
| Example 1-11 | 2800 | 0.60 | 490 | 1.56 | Uncomfortable (35) |
| Example 1-12 | 2800 | 0.70 | 490 | 1.50 | Feel like uncomfortable (43) |
| Example 1-13 | 4250 | 0.95 | 490 | 1.35 | Not uncomfortable (38) |
| Example 1-14 | 4000 | 0.70 | 470 | 1.20 | Not uncomfortable (33) |
| Example 1-15 | 5000 | 0.90 | 455 | 1.15 | Comfortable (32) |
| Example 1-16 | 5200 | 0.70 | 470 | 1.12 | Comfortable (40) |
| Example 1-17 | 6500 | 0.85 | 450 | 1.10 | Comfortable (38) |
| Example 1-18 | 3000 | 0.85 | 490 | 1.10 | Comfortable (43) |
| Example 1-19 | 2800 | 0.98 | 490 | 1.05 | Comfortable (48) |
| Example 1-20 | 2900 | 0.90 | 490 | 0.95 | Comfortable (51) |
| Comparative Example 1-3 | 5000 | 0.30 | 450 | 2.47 | Very uncomfortable (36) |
| Comparative Example 1-4 | 2800 | 0.20 | 448 | 2.03 | Uncomfortable (52) |

From the results in Table 3, in the sensory test of the white light emitting devices, it was judged to be obviously uncomfortable when the intensity ratio (A*/ B*) is over 1.5, the uncomfortable feeling gradually decreased as the numeric value decreased at 1.5 or less, when the intensity ratio became approximately 1.0, the number of subjects evaluating to be comfortable increased. As stated above, it turns out that the light source becomes fine one which does not make someone feel uncomfortable when the intensity of the blue component is close to or lower than the spectrum of the black body radiation. Besides, relative color temperatures of the white lights of the example 1-10 to the example 1-20 were 2500 K or more and 7000 K or less.

### (Examples 1-1B to 1-20B, Comparative Example 1-1B)

Lighting systems were manufactured by each using 20 pieces of white light emitting devices of the example 1-1 to the example 1-20 and the comparative example 1-1 (an example 1-1B to an example 1-20B, a comparative example 1-1B). Each of the lighting systems was used to irradiate at a dental filling composite resin at a light intensity of 5000 lx, and a time until the resin was cured was measured. Measurement results are illustrated in Table 4.

**[Table 4]**

| | RESIN CURING TIME |
|---|---|
| EXAMPLE 1-1B | 7 minutes 26 seconds |
| EXAMPLE 1-2B | 7 minutes 24 seconds |
| EXAMPLE 1-3B | 7 minutes 53 seconds |
| EXAMPLE 1-4B | 6 minutes 20 seconds |
| EXAMPLE 1-5B | 3 minutes 57 seconds |
| EXAMPLE 1-6B | 3 minutes 48 seconds |
| EXAMPLE 1-7B | 3 minutes 45 seconds |
| EXAMPLE 1-8B | 8 minutes 10 seconds |
| EXAMPLE 1-9B | 4 minutes 32 seconds |
| EXAMPLE 1-10B | 3 minutes 40 seconds |
| EXAMPLE 1-11B | 4 minutes 53 seconds |
| EXAMPLE 1-12B | 5 minutes 12 seconds |
| EXAMPLE 1-13B | 3 minutes 40 seconds |
| EXAMPLE 1-14B | 3 minutes 43 seconds |
| EXAMPLE 1-15B | 3 minutes 35 seconds |
| EXAMPLE 1-16B | 3 minutes 33 seconds |
| EXAMPLE 1-17B | 3 minutes 23 seconds |
| EXAMPLE 1-18B | 3 minutes 28 seconds |
| EXAMPLE 1-19B | 6 minutes 22 seconds |
| EXAMPLE 1-20B | 6 minutes 09 seconds |
| COMPARATIVE EXAMPLE 1-1B | 1 minutes 58 seconds |

From Table 4, it can be seen that the curing time of the dental resin is long in each of the lighting systems according to the example 1-1B to the example 1-20B compared to the lighting system according to the comparative example 1-1B. As stated above, it is possible for the lighting systems according to the present example to make the curing time of the dental resin long, and therefore, it is possible to prevent the early curing of the dental resin more than necessary in the dental treatment.

### <Example 2>

In the present example, concrete examples of the white light emitting device according to the second embodiment are described.

### (Example 2-1)

An LED chip having a light emission peak wavelength of 405 nm was prepared, and disposed on an alumina substrate. Next, the LED chip was covered with a silicone resin being a transparent resin to form a transparent resin layer.

Next, as phosphors, a europium-activated alkaline earth halophosphate blue phosphor having a light emission peak wavelength of 445 nm, a europium-activated orthosilicate green phosphor having a light emission peak wavelength of 520 nm, a europium-activated orthosilicate yellow phosphor having a light emission peak wavelength of 540 nm, and a europium-activated strontium sialon red phosphor having a light emission peak wavelength of 630 nm were prepared. Note that an average grain diameter of each phosphor was approximately 10 µm. Further, respective phosphors were mixed at a ratio of the blue phosphor: the green phosphor: the yellow phosphor: the red phosphor = 93.0: 1.5: 2.0: 3.5 as a weight ratio (mass ratio), then mixed with the transparent resin (silicone resin), and a phosphor layer was provided on the transparent resin layer to thereby manufacture a white light emitting device. A correlated color temperature of an emission color of the white light emitting device was 4780 K. Besides, a thickness of the phosphor layer was set to be 0.3 mm.

Among the above-stated phosphors, half value widths of the light emission peak wavelengths of the blue phosphor, the green phosphor, the yellow phosphor, and the red phosphor were each 50 nm or more. Besides, in light emission spectra of the blue phosphor, the green phosphor, the yellow phosphor, and the red phosphor, a region where the light emission spectra of the phosphors whose light emission peak wavelengths were adjacent were overlapped was each held.

Next, a light emission spectrum of the white light emitting device of the example 2-1 was measured by the total luminous flux measurement using the integrating sphere. The measurement result is illustrated in Fig. 13. Further, light of the white light emitting device was lighted on a dental filling composite resin at a light intensity of 5000 lx, and a time until the resin was cured was measured.

### (Example 2-2)

An LED chip having a light emission peak wavelength of 405 nm was prepared, and disposed on an alumina substrate. Next, the LED chip was covered with a silicone resin being a transparent resin to form a transparent resin layer.

Next, as phosphors, a europium-activated alkaline earth halophosphate blue phosphor having a light emission peak wavelength of 445 nm, a europium-activated orthosilicate green phosphor having a light emission peak wavelength of 520 nm, a europium-activated orthosilicate yellow phosphor having a light emission peak wavelength of 540 nm, and a europium-activated strontium sialon red phosphor having a light emission peak wavelength of 630 nm were prepared. Note that an average grain diameter of each phosphor was approximately 18 µm. Further, respective phosphors were mixed at a ratio of the blue phosphor: the green phosphor: the yellow phosphor: the red phosphor = 93.4: 1.7: 1.5: 3.4 as a weight ratio (mass ratio), then mixed with the transparent resin (silicone resin), and a phosphor layer was provided on the transparent resin layer to thereby manufacture a white light emitting device. A correlated color temperature of an emission color of the white light emitting device was 5360 K. Besides, a thickness of the phosphor layer was set to be 0.5 mm.

Among the above-stated phosphors, half value widths of the light emission peak wavelengths of the green phosphor, the yellow phosphor, and the red phosphor were each 50 nm or more. Besides, in light emission spectra of the blue phosphor, the green phosphor, the yellow phosphor, and the red phosphor, a region where the light emission spectra of the phosphors whose light emission peak wavelengths were adjacent were overlapped was each held.

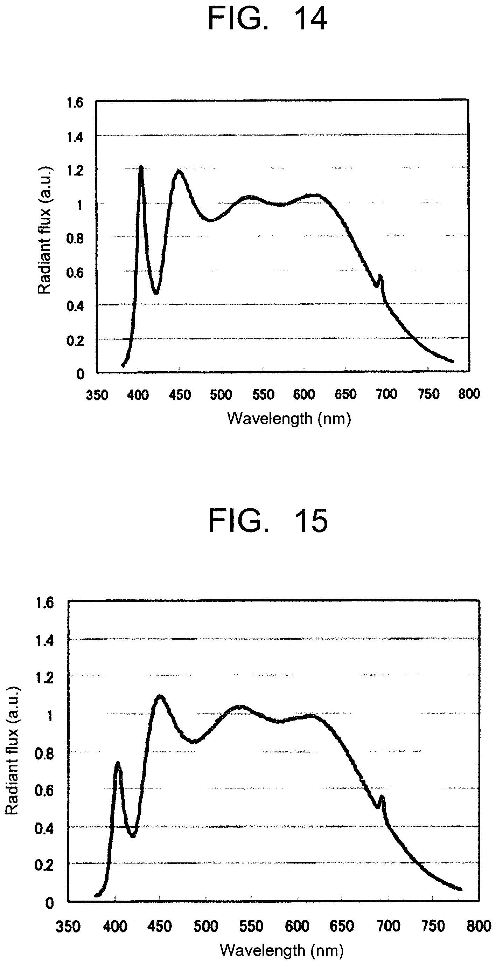

Next, a light emission spectrum of the white light emitting device of the example 2-2 was measured by the total luminous flux measurement using the integrating sphere as same as the example 2-1. The measurement result is illustrated in Fig. 14. Further, a resin curing time of a dental filling composite resin was measured as same as the example 2-1.

### (Example 2-3)

An LED chip having a light emission peak wavelength of 405 nm was prepared, and disposed on an alumina substrate. Next, the LED chip was covered with a silicone resin being a transparent resin to form a transparent resin layer.

Next, as phosphors, a europium-activated alkaline earth halophosphate blue phosphor having a light emission peak wavelength of 445 nm, a europium-activated β sialon green phosphor having a light emission peak wavelength of 530 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 18 µm. Further, respective phosphors were mixed at a ratio of the blue phosphor: the green phosphor: the yellow phosphor: the red phosphor = 92.9: 2.5: 1.5: 3.1 as a weight ratio (mass ratio), then mixed with the transparent resin (silicone resin), and a phosphor layer was provided on the transparent resin layer to thereby manufacture a white light emitting device. A correlated color temperature of an emission color of the white light emitting device was 5290 K. Besides, a thickness of the phosphor layer was set to be 0.5 mm.

Among the above-stated phosphors, half value widths of the light emission peak wavelengths of the green phosphor, the yellow phosphor, and the red phosphor were each 50 nm or more. Besides, in light emission spectra of the blue phosphor, the green phosphor, the yellow phosphor, and the red phosphor, a region where the light emission spectra of the phosphors whose light emission peak wavelengths were adjacent were overlapped was each held.

Next, a light emission spectrum of the white light emitting device of the example 2-3 was measured by the total luminous flux measurement using the integrating sphere as same as the example 2-1. The measurement result is illustrated in Fig. 15.

Besides, a resin curing time of a dental filling composite resin was measured as same as the example 2-1.

### (Example 2-4)

An LED chip having a light emission peak wavelength of 405 nm was prepared, and disposed on an alumina substrate. Next, the LED chip was covered with a silicone resin being a transparent resin to form a transparent resin layer.

Next, as phosphors, a europium-activated alkaline earth halophosphate blue phosphor having a light emission peak wavelength of 445 nm, a europium-activated orthosilicate yellow phosphor having a light emission peak wavelength of 530 nm, a europium-activated orthosilicate yellow phosphor having a light emission peak wavelength of 555 nm, and a europium-activated strontium sialon red phosphor having a light emission peak wavelength of 630 nm were prepared. Note that an average grain diameter of each phosphor was approximately 18 µm. Further, respective phosphors are mixed at a ratio of the blue phosphor: the green phosphor: the yellow phosphor: the red phosphor = 91.7: 2.2: 2.1: 4.0 as a weight ratio (mass ratio), then mixed with the transparent resin (silicone resin), and a phosphor layer was provided on the transparent resin layer to thereby manufacture a white light emitting device. A correlated color temperature of an emission color of the white light emitting device of the example 2-4 was 4040 K. Besides, a thickness of the phosphor layer was set to be 0.5 mm.

Among the above-stated phosphors, half value widths of the light emission peak wavelengths of the green phosphor, the yellow phosphor, and the red phosphor were each 50 nm or more. Besides, in light emission spectra of the blue phosphor, the green phosphor, the yellow phosphor, and the red phosphor, a region where the light emission spectra of the phosphors whose light emission peak wavelengths were adjacent were overlapped was each held.

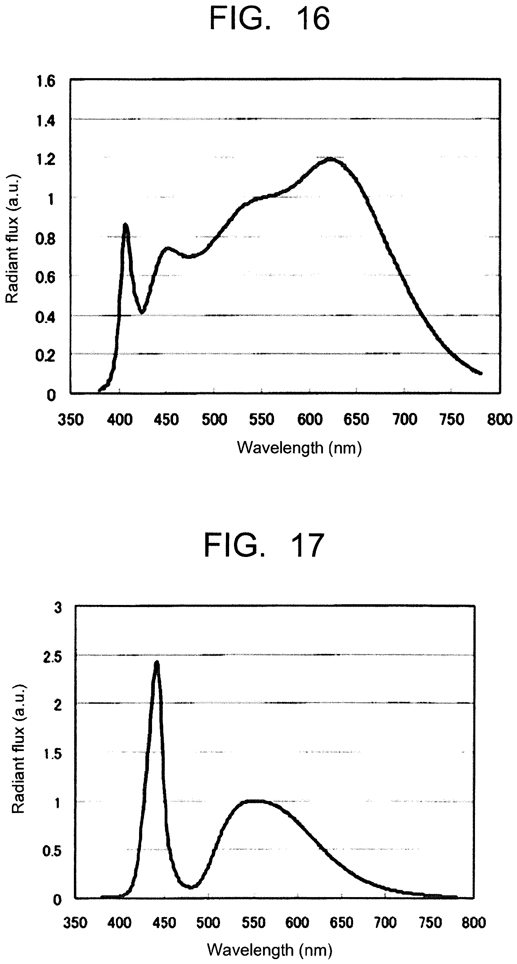

Next, a light emission spectrum of the white light emitting device of the example 2-4 was measured by the total luminous flux measurement using the integrating sphere as same as the example 2-1. The measurement result is illustrated in Fig. 16. Besides, a resin curing time of a dental filling composite resin was measured as same as the example 2-1.

### (Comparative Examples 2-1 to 2-3)

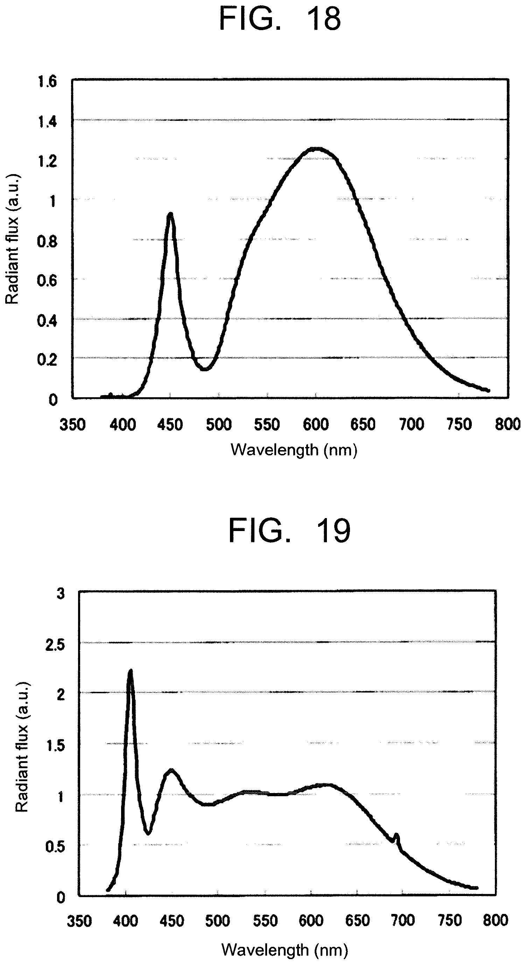

White light emitting devices of a comparative example 2-1 to a comparative example 2-3 were prepared by changing the light emission peak wavelength of the light-emitting diode, kinds of the phosphors, and a content of the phosphor. Note that a light emission spectrum of the white light emitting device of the comparative example 2-1 is illustrated in Fig. 17, a light emission spectrum of the white light emitting device of the comparative example 2-2 is illustrated in Fig. 18, and a light emission spectrum of the white light emitting device of the comparative example 2-3 is illustrated in Fig. 19.

In the white light emitting devices of the example 2-1 to the example 2-4 and the comparative examples 2-1 to 2-3, an integral value A (a radiant flux integral value at 380 nm or more and 780 nm or less), an integral value B (a radiant flux integral value at 380 nm or more and 480 nm or less), an integral value C (a radiant flux integral value at 380 nm or more and 429 nm or less), and an integral value D (a radiant flux integral value at 430 nm or more and 480 nm or less) were measured, and the integral value B/ the integral value A, the integral value C/ the integral value A, and the integral value D/ the integral value A were found. Similarly, a peak radiant flux ratio at a range of 430 nm or more and 480 nm or less and a peak radiant flux ratio at a range of 380 nm or more and 420 nm or less relative to a peak radiant flux of a light emission spectrum at 555 nm were each found. The results are illustrated in Table 5.

**[Table 5]**

| | Integral value B/ integral value A | Integral value C/ integral value A | Integral value D/ integral value A | (peak₄₃₀₋₄₈₀ₙₘ/ peak₅₅₅ₙₘ) | (peak₃₈₀₋₄₂₀ₙₘ/ peak₅₅₅ₙₘ) |
|---|---|---|---|---|---|
| Example 2-1 | 0.22 | 0.05 | 0.16 | 1.04 | 0.57 |
| Example 2-2 | 0.27 | 0.09 | 0.18 | 1.19 | 1.21 |
| Example 2-3 | 0.24 | 0.07 | 0.12 | 1.10 | 0.74 |
| Example 2-4 | 0.19 | 0.07 | 0.12 | 0.74 | 0.86 |
| Comparative Example 2-1 | 0.31 | 0.05 | 0.26 | 2.43 | 0.39 |
| Comparative Example 2-2 | 0.12 | 0.01 | 0.11 | 0.92 | 0.05 |
| Comparative Example 2-3 | 0.31 | 0.14 | 0.17 | 1.24 | 2.22 |

In the white light emitting devices of the example 2-1 to the example 2-4 and the comparative example 2-1 to the comparative example 2-3, the average color rendering index Ra, the special color rendering index R9 were also examined. Further, presence/absence of sensation of glare of a radiated light, a blood vessel color (how the blood vessel looks) at a palm of a hand, and a contrast between white of JIS color sample and sunlight in daytime (how white looks) were illustrated in Table 6. Note that when "there is" the sensation of glare, it is felt glare more than necessary. Besides, the resin curing time of the dental filling composite resin was also illustrated.

**[Table 6]**

| | Correlated color temperature (k) | Average color rendering index Ra | Special color rendering index R9 | Resin curing time | Sensation of glare | how blood vessel looks | how white looks |
|---|---|---|---|---|---|---|---|
| Example 2-1 | 4760 | 98.3 | 92 | 3m 50s | Absent | Look natural | Look natural |
| Example 2-2 | 5360 | 97.5 | 94 | 3m 10s | Absent | Look natural | Look natural |
| Example 2-3 | 5290 | 97.5 | 86 | 3m 30s | Absent | Look natural | Look natural |
| Example 2-4 | 4040 | 98.4 | 90 | 4m 45s | Absent | Look almost natural | Slightly yellow-tinged |
| Comparative Example 2-1 | 6239 | 67.1 | -21 | 1m 59s | Present | Red is unnatural | Unnatural |
| Comparative Example 2-2 | 3110 | 78.9 | 17 | 7m 30s | Absent | Red is too strung | Red-tinged |
| Comparative Example 2-3 | 5373 | 95.4 | 94 | 2m 9s | Present | Look natural | Look natural |

As it can be seen from Table 6, in the white light emitting devices according to the example, it turns out that the early curing of the dental filling composite resin is suppressed. Besides, the average color rendering index Ra, the special color rendering index R9 are each within a predetermined range, and therefore, there is not the sensation of glare, and it is not felt glare more than necessary. Besides, the way how the blood vessel looks and the way how white looks are natural, and therefore, it turns out that it shows an excellent color rendering property in a lighting to light up not only in the mouth but also any of the task lighting, the printed matter, the food material, and the person, and in a lighting used at a close place such as 1.5 m or less to an object to be lighted.

## Claims

1. A white light emitting device adapted to a dental lighting system, the white light emitting device comprising:
a light-emitting diode emitting first light having a light emission intensity peak at a wavelength region of from 350 to 490 nm; and
a phosphor layer emitting white light in response to excitation by the first light of the light-emitting diode,
wherein
the phosphor layer includes a plurality of phosphors which emit second lights by being excited by the first light, the second lights have light emission intensity peaks different from one another at a wavelength region of from 420 to 700 nm, and the white light is formed by the second lights, or the first light and the second lights,
among a light emission spectrum of the light-emitting diode and each of the light emission spectra of the plurality of phosphors, it has at least one region where two or more light emission spectra are overlapped,
a relative color temperature of the white light is 2500 K or more and 7000 K or less, and
in a light emission spectrum of the white light, when an arbitrary local maximum value of a light emission intensity of the local maximum values existing in the wavelength region of from 350 to 780 nm of the white light is set to be "1", and a ratio of a local minimum value of the light emission intensity which is in closest proximity to said arbitrary local maximum value at a long wavelength side relative to said arbitrary local maximum value is set to an intensity ratio, all of the intensity ratios are 0.5 or more.

2. The white light emitting device adapted to a dental lighting system according to claim 1,
wherein, when a light emission spectrum of the white light and a light emission spectrum of a black body radiation whose color temperature is the same as the white light are compared under an identical visual luminance, a ratio of a maximum light emission intensity of the white light at a wavelength region of from 380 to 490 nm relative to a light emission intensity of the black body radiation is 1.5 or less.

3. The white light emitting device adapted to a dental lighting system according to claim 1,
wherein the white light has a maximum peak intensity at a wavelength region of more than 490 nm and 780 nm or less.

4. The white light emitting device adapted to a dental lighting system according to claim 1,
wherein a half value width of at least one of the light emission intensity peaks of the plurality of phosphors is 50 nm or more and 100 nm or less.

5. A dental lighting system comprising the white light emitting device as defined in claim 1.

## Patentansprüche

1. Weißlichtemittierende Vorrichtung, die an ein dentales Beleuchtungssystem angepasst ist, wobei die weißlichtemittierende Vorrichtung umfasst:
eine lichtemittierende Diode, die ein erstes Licht mit einem Lichtemissionsintensitätspeak im Wellenlängenbereich von 350 bis 490 nm emittiert; und
eine Leuchtstoffschicht, die als Reaktion auf die Anregung durch das erste Licht der lichtemittierenden Diode weißes Licht emittiert,
wobei
die Leuchtstoffschicht eine Mehrzahl von Leuchtstoffen enthält, die durch Anregung mit dem ersten Licht zweite Lichter emittieren, wobei die zweiten Lichter voneinander verschiedene Lichtemissionsintensitätspeaks im Wellenlängenbereich von 420 bis 700 nm aufweisen und das weiße Licht durch die zweiten Lichter oder das erste Licht und die zweiten Lichter gebildet wird,
unter einem Lichtemissionsspektrum der lichtemittierenden Diode und jedem der Lichtemissionsspektren der Mehrzahl von Leuchtstoffen mindestens ein Bereich vorhanden ist, in dem sich zwei oder mehr Lichtemissionsspektren überlappen,
eine relative Farbtemperatur des weißen Lichts 2500 K oder mehr und 7000 K oder weniger beträgt, und
in einem Lichtemissionsspektrum des weißen Lichts, wenn ein willkürlicher lokaler Maximalwert einer Lichtemissionsintensität der lokalen Maximalwerte, die im Wellenlängenbereich von 350 bis 780 nm des weißen Lichts existieren, als "1" festgelegt wird, und ein Verhältnis eines lokalen Minimalwerts der Lichtemissionsintensität, der in nächster Nähe zu dem willkürlichen lokalen Maximalwert auf der Seite zu langen Wellenlängen relativ zu dem willkürlichen lokalen Maximalwert liegt, als ein Intensitätsverhältnis festgelegt wird, alle Intensitätsverhältnisse 0,5 oder mehr sind.

2. Weißlichtemittierende Vorrichtung, angepasst an ein dentales Beleuchtungssystem, gemäß Anspruch 1,
wobei, wenn ein Lichtemissionsspektrum des weißen Lichts und ein Lichtemissionsspektrum einer Schwarzkörperstrahlung, deren Farbtemperatur die gleiche wie die des weißen Lichts ist, unter einer identischen visuellen Luminanz verglichen werden, ein Verhältnis von einer maximalen Lichtemissionsintensität des weißen Lichts im Wellenlängenbereich von 380 bis 490 nm relativ zu einer Lichtemissionsintensität der Schwarzkörperstrahlung 1,5 oder weniger ist.

3. Weißlichtemittierende Vorrichtung, angepasst an ein dentales Beleuchtungssystem, gemäß Anspruch 1,
wobei das weiße Licht eine maximale Peakintensität im Wellenlängenbereich von mehr als 490 nm und 780 nm oder weniger aufweist.

4. Weißlichtemittierende Vorrichtung, angepasst an ein dentales Beleuchtungssystem, gemäß Anspruch 1,
wobei eine Halbwertsbreite von mindestens einem der Lichtemissionsintensitätspeaks der Mehrzahl von Leuchtstoffen 50 nm oder mehr und 100 nm oder weniger beträgt.

5. Dentales Beleuchtungssystem, umfassend die weißlichtemittierende Vorrichtung, wie in Anspruch 1 definiert.

## Revendications

1. Dispositif d'émission de lumière blanche adapté à un système d'éclairage dentaire, le dispositif d'émission de lumière blanche comprenant :
une diode électroluminescente émettant une première lumière présentant un pic d'intensité d'émission de lumière au niveau d'une région de longueur d'onde de 350 à 490 nm ; et
une couche de luminophore émettant une lumière blanche en réponse à une excitation par la première lumière de la diode électroluminescente,
dans lequel
la couche de luminophore inclut une pluralité de luminophores qui émettent des secondes lumières en étant excités par la première lumière,
les secondes lumières présentent des pics d'intensité d'émission de lumière différents les uns des autres au niveau d'une région de longueur d'onde de 420 à 700 nm,
et la lumière blanche est formée par les secondes lumières, ou la première lumière et les secondes lumières,
parmi un spectre d'émission de lumière de la diode électroluminescente et chacun des spectres d'émission de lumière de la pluralité de luminophores, il présente au moins une région où deux spectres d'émission de lumière ou plus se chevauchent,
une température de couleur relative de la lumière blanche est de 2500 K ou plus et 7000 K ou moins, et
dans un spectre d'émission de lumière de la lumière blanche, lorsqu'une valeur maximale locale arbitraire d'une intensité d'émission de lumière des valeurs maximales locales existant dans la région de longueur d'onde de 350 à 780 nm de la lumière blanche est réglée pour être "1", et qu'un rapport d'une valeur minimale locale de l'intensité d'émission de lumière qui est en proximité la plus proche de ladite valeur maximale locale arbitraire au niveau d'un côté longue longueur d'onde par rapport à ladite valeur maximale locale arbitraire est réglé sur un rapport d'intensité, tous les rapports d'intensité sont de 0,5 ou plus.

2. Dispositif d'émission de lumière blanche adapté à un système d'éclairage dentaire selon la revendication 1,
dans lequel, lorsqu'un spectre d'émission de lumière de la lumière blanche et un spectre d'émission de lumière d'un rayonnement de corps noir dont la température de couleur est la même que celle de la lumière blanche sont comparés sous une luminance visuelle identique, un rapport d'une intensité d'émission de lumière maximale de la lumière blanche au niveau d'une région de longueur d'onde de 380 à 490 nm par rapport à une intensité d'émission de lumière du rayonnement de corps noir est de 1,5 ou moins.

3. Dispositif d'émission de lumière blanche adapté à un système d'éclairage dentaire selon la revendication 1,
dans lequel la lumière blanche présente une intensité de pic maximale au niveau d'une région de longueur d'onde de plus de 490 nm et 780 nm ou moins.

4. Dispositif d'émission de lumière blanche adapté à un système d'éclairage dentaire selon la revendication 1,
dans lequel une largeur de demi-valeur d'au moins un des pics d'intensité d'émission de lumière de la pluralité de luminophores est de 50 nm ou plus et de 100 nm ou moins.

5. Système d'éclairage dentaire comprenant le dispositif d'émission de lumière blanche tel que défini dans la revendication 1.
